# EUROPÄISCHE PATENTSCHRIFT

(11) **EP 1 195 716 B1**
(45) Veröffentlichungstag und Bekanntmachung des Hinweises auf die Patenterteilung: **31.01.2018**
(21) Anmeldenummer: 01123632.0
(22) Anmeldetag: 02.10.2001
(51) Int. Cl.: A61B 6/02, G06T 11/00

(54) **Computertomograph mit kegelförmigem Strahlenbündel und helixförmiger Relativbewegung**
Computer tomograph with conical beam and helical relative motion
Tomographe assisté par ordinateur avec un faisceau de rayonnement conique et un mouvement relatif hélicoidal

(30) Priorität: 05.10.2000 DE 10049380
(43) Veröffentlichungstag der Anmeldung: 10.04.2002
(73) Patentinhaber: Philips Intellectual Property & Standards GmbH, 20099 Hamburg (DE); Koninklijke Philips N.V., 5656 AE Eindhoven (NL)
(72) Erfinder: Köhler, Thomas, Dr., 52064 Aachen (DE); Grass, Michael, Dr., 52064 Aachen (DE)
(74) Vertreter: de Haan, Poul Erik

(56) Entgegenhaltungen:
- EP-A- 0 383 232
- EP-A- 0 428 348
- JP-A- 2000 116 647

## Beschreibung

Die Erfindung betrifft einen Computertomographen mit einer ein kegelförmiges Strahlenbündel emittierenden Strahlenquelle, die sich relativ zu einem Untersuchungsbereich bzw. einem darin befindlichen Objekt auf einer helixförmigen Bahn bewegt. Bei Computertomographen dieser Art - die z. B. aus der Patentveröffentlichung US 5233518 A bekannt sind - kann die Absorption in den Bereichen, die zu Beginn oder am Ende einer CT-Untersuchung von dem kegelförmigen Strahlenbündel erfasst werden, nicht frei von Artefakten rekonstruiert werden, weil diese Bereiche von der Strahlenquelle nur aus einem begrenzten Winkelbereich bestrahlt werden.
Das Verhältnis zwischen dem von Strahlung getroffenen Bereich und dem artefaktfrei rekonstruierbaren Bereich ist umso ungünstiger, je größer der Öffnungswinkel des Strahlenbündels (in zwei zueinander senkrechten Ebenen) ist. Es kann also nur ein Teil der Strahlung für die CT-Bildgebung ausgenutzt werden, was bei medizinischen Untersuchungen zu einer unnötig hohen Strahlenbelastung des Patienten führt. Wenn man darüber hinaus den zylinderförmigen Untersuchungsbereich mit der ersten bzw. der letzten vollständig rekonstruierbaren und zum zylinderförmigen Untersuchungsbereich senkrechten Schicht beginnen bzw. enden lässt, werden die Verhältnisse vollends ungünstig.
Aufgabe der vorliegenden Erfindung ist es daher, diese Verhältnisse zu verbessern. Diese Aufgabe wird erfindungsgemäß gelöst durch einen Computertomographen gemäß Anspruch 1.
Die Erfindung beruht auf der Erkenntnis, dass der zylinderförmige Teil des Untersuchungsbereichs, für den eine vollständige Rekonstruktion möglich ist, beiderseits durch Stirnflächen begrenzt ist, die zur Rotationsachse nicht senkrecht verlaufen und auch nicht eben sind. Durch geeignete Wahl der Startposition und/oder der Stoppposition lässt sich nun erreichen, dass - in Richtung der Rotationsachse gemessen - der rekonstruierbare Teil des Untersuchungsbereichs oberhalb der Lagerungsplatte größer ist als unterhalb (bzw. dass der nicht rekonstruierbare Teil unterhalb der Lagerungsplatte größer ist als oberhalb).

Der durch zur Rotationsachse senkrechte Schichten darstellbare Bereich kann dann entsprechend den Abmessungen des rekonstruierbaren Bereichs oberhalb der Lagerungsplatte gewählt werden. In diesem Fall kann in den am Rande liegenden Schichten der Bereich unterhalb der Lagerungsplatte zwar nicht mehr rekonstruiert werden, doch ist dies irrelevant, weil sich das Objekt und damit die für die Diagnose relevanten Bereiche oberhalb der Lagerungsplatte befinden.
Die optimale Start- bzw. Stoppposition der Strahlenquelle befindet sich in der Regel oberhalb einer horizontalen, die Rotationsachse enthaltenden Ebene. Die genaue Start-bzw. Stoppposition hängt aber auch davon ab, wie die Messwerte akquiriert werden und wie aus diesen Messwerten ein dreidimensionales CT-Bild rekonstruiert wird.

Da bei den bekannten Verfahren die Stirnflächen des vollständig rekonstruierbaren Bereiches nicht eben sind, hängt die optimale Startposition auch davon ab, in welcher Höhe die Lagerungsplatte diese Stirnflächen durchstößt. Befindet sich die Lagerungsplatte bei einer CT-Untersuchung beispielsweise unterhalb der Rotationsachse, dann müssen die Startposition bzw. die Stopposition in Umlaufrichtung jeweils um denselben Winkelbetrag versetzt werden. Anspruch 2 beschreibt nun eine Ausgestaltung, mit der die Start- bzw. die Stopposition automatisch der vertikalen Position der Lagerungsplatte angepasst werden kann.
Wie bereits erwähnt, lassen sich in den an den Enden befindlichen Schichten die Bereiche unterhalb der Lagerungsplatteplatte nicht mehr vollständig rekonstruieren, so dass sich dort Artefakte ergeben. Diese können gemäß Anspruch 3 vermieden werden, wobei der Untersucher den gewohnten Bildeindruck erhält. Man könnte die nicht vollständig rekonstruierbaren Bereiche unterhalb der Lagerungsplatte bei der Wiedergabe aber auch auf geeignete Weise markieren (z.B. farbig), um dem Benutzer nicht eine vollständige Rekonstruktion in diesem Bereich vorzuspiegeln.

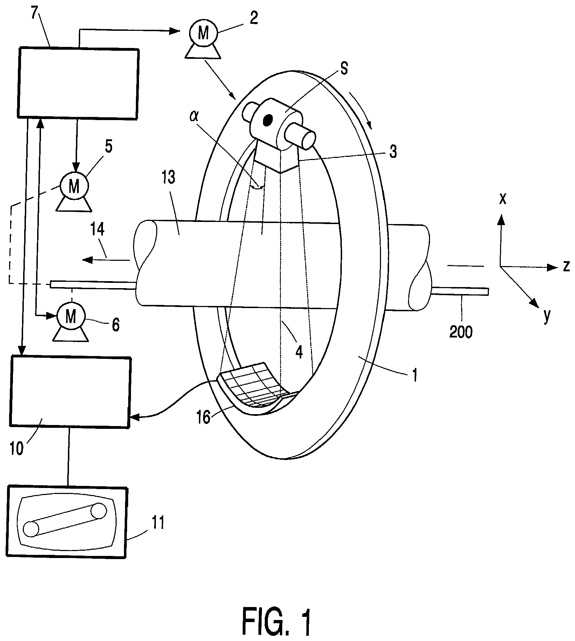

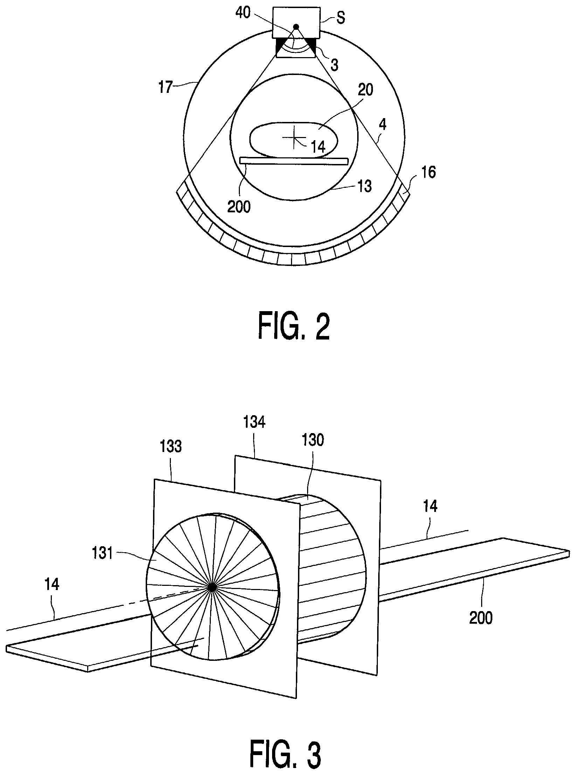

Die Erfindung wird nachstehend anhand der Zeichnungen näher erläutert. Es zeigen
Fig. 1 einen erfindungsgemäßen Computertomographen in schematischer und perspektivischer Darstellung,
Fig. 2 einen Querschnitt durch einen solchen Computertomographen,
Fig. 3 den rekonstruierbaren Teil des Untersuchungsbereichs ohne die Erfindung,
Fig. 4 a und Fig. 4b die Start- bzw. die Stopposition der Strahlenquelle,
Fig. 5 den rekonstruierbaren Teil des Untersuchungsbereichs mit der Erfindung,
Fig. 6 a und Fig. 6b die Stirnflächen des rekonstruierbaren Bereichs.
Der in den Fig. 1 und 2 dargestellte Computertomograph umfasst eine Gantry 1, die um eine parallel zur z-Richtung des in Fig. 1 dargestellten Koordinatensystems verlaufende (zur Zeichenebene der Fig. 2 senkrechte) Rotationsachse 14 rotieren kann. Dazu wird die Gantry von einem Motor 2 mit einer vorzugsweise konstanten, aber einstellbaren Geschwindigkeit angetrieben. An der Gantry ist eine Strahlenquelle S befestigt, beispielsweise ein Röntgenstrahler. Dieser ist mit einer Kollimatoranordnung 3 verbunden, die aus der von der Strahlenquelle S erzeugten Strahlung unter anderem mittels einer Blendenanordnung 40 ein kegelförmiges Strahlenbündel ausblendet, d. h. ein Strahlenbündel, das sowohl in z-Richtung als auch in einer dazu senkrechten Richtung (d. h. in einer zur Rotationsachse 14 senkrechten Ebene) eine von Null verschiedene endliche Ausdehnung hat.

Das Strahlenbündel 4 durchdringt einen Untersuchungsbereich 13, in dem sich ein Objekt 20, z. B. ein Patient auf einer Lagerungsplatte 200 eines im übrigen nicht näher dargestellten Patientenlagerungstisches, befinden kann. Der Untersuchungsbereich 13 hat die Form eines Zylinders, dessen Durchmesser durch den Öffnungswinkel α des Strahlenbündels 4 (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse definierten Ebene einschließt) bestimmt ist.

Nach dem Durchsetzen des Untersuchungsbereichs 13 trifft das Röntgenstrahlenbündel 4 auf eine an der Gantry 1 befestigte zweidimensionale Detektoreinheit 16, die eine Anzahl von nebeneinander angeordneten Detektorzeilen mit jeweils einer Vielzahl von Detektorelementen umfasst. Die Detektorzeilen befinden sich in zur Rotationsachse 14 senkrechten Ebenen, vorzugsweise auf einem Kreisbogen um die Strahlenquelle S. Sie können aber auch anders geformt sein, z. B. einen Kreisbogen um die Rotationsachse 14 beschreiben oder geradlinig sein. Jedes von dem Strahlenbündel 4 getroffene Detektorelement liefert in jeder Position der Strahlungsquelle einen Messwert für einen Strahl aus dem Strahlenbündel 4. Die Messwerte werden im folgenden auch als CT-Daten bezeichnet.

Der Untersuchungsbereich 13 bzw. die Lagerungsplatte 200 kann mittels eines Motors 5 parallel zur Rotationsachse 14 bzw. parallel zur z-Achse verschoben werden. Stattdessen könnte aber auch die Gantry in der Gegenrichtung verschoben werden. Es kommt nämlich sowohl bei der Rotation der Strahlenquelle um die Rotationsachse 14 als auch bei der Verschiebung des Untersuchungsbereichs in Richtung der Rotationsachse 14 nur auf die Relativbewegung zwischen der Gantry 1 und dem Untersuchungsbereich 13 an. Wenn die Motoren 5 und 2 gleichzeitig laufen, beschreiben die Strahlungsquelle S und die Detektoreinheit 16 eine Trajektorie in Form einer Helix relativ zum Untersuchungsbereich 13. Wenn hingegen der Motor 5 für den Vorschub in z-Richtung stillsteht und der Motor 2 die Gantry 1 rotieren lässt, ergibt sich eine kreisförmige Trajektore für die Strahlenquelle S und die Detektoreinheit 16 relativ zum Untersuchungsbereich 13.

Schließlich kann mittels eines Motors 6 die Lagerungsplatte 200 in der Höhe verstellt werden, wobei gleichzeitig die aktuelle Höhe der Lagerungsplatte gemessen und einer Steuereinheit 7 zugeführt wird. Die Steuereinheit 7 steuert darüber hinaus die Motoren 2, 5 und 6, die Strahlenquelle S sowie den Transfer der CT-Daten von der Detektoreinheit 16 zu einem Bildverarbeitungsrechner 10. In dem Bildverarbeitungsrechner 10 wird aus den CT-Daten ein CT-Bild rekonstruiert, das die Absorption in dem (dreidimensionalen) Teil des Untersuchungsbereichs darstellt, für den hinreichend CT-Daten akquiriert werden können.

In Fig. 3 ist dieser Teil des Untersuchungsbereichs mit 130 bezeichnet. Sein Querschnitt in einer zur Rotationsachse 14 senkrechten Ebene entspricht dem Querschnitt des Untersuchungsbereichs 13. Der rekonstruierbare Bereich 130 wird beiderseits durch Stirnflächen 131 und 132 (die hintere Stirnfläche 132 ist in Fig. 3 nicht erkennbar) begrenzt. Die Stirnflächen verlaufen aber nicht senkrecht zur Rotationsachse, und sie sind auch nicht eben. Infolge dessen wird man nur den Teil des Bereichs 130 darstellen (und rekonstruieren), der in zur Rotationsachse senkrechten Schichten vollständig rekonstruiert werden kann. Dieser Teil wird beiderseits durch die zur Rotationsachse 14 senkrechten Ebenen 133 und 134 begrenzt. Man erkennt, dass der z. B. zwischen der Ebene 133 und der Stirnfläche 131 liegende Teil verloren geht, in welchem die Schichten nur einen Teil des Querschnitts des Untersuchungsbereichs 13 darstellen würden.

Bei der Erfindung werden nun die Start- bzw. Stopposition der Strahlenquelle, in der die Relativbewegung zwischen Strahlenquelle und Untersuchungsbereich und die Emission des Strahlenbündels beginnt bzw. endet, so gewählt, dass die nicht mehr rekonstruierbaren Zonen des Untersuchungsbereiches innerhalb oder unterhalb der Lagerungsplatte 200 liegen. Die für die Diagnose relevanten Zonen in der Schicht oberhalb der Lagerungsplatte, wo sich das Objekt befindet, sind hingegen vollständig rekonstruierbar.

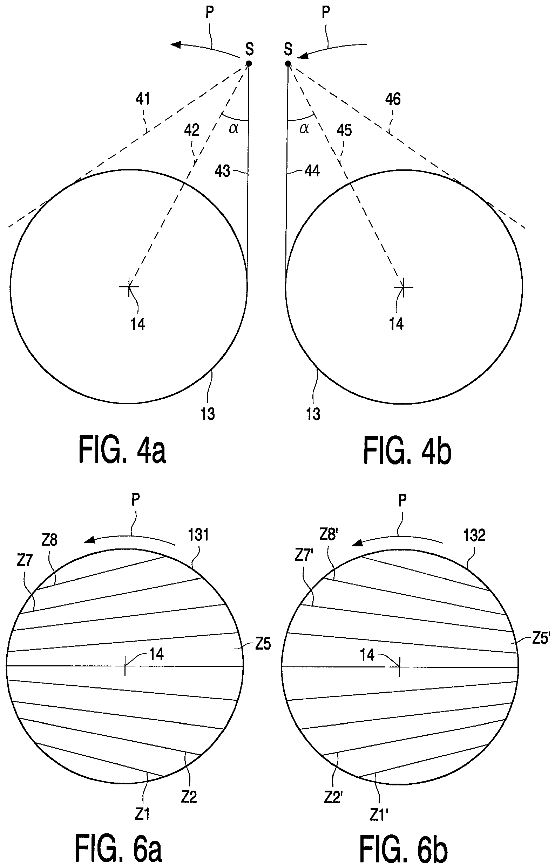

Dies wird im einzelnen anhand der Fig. 4 a und 4 b erläutert, die die Strahlenquelle S in der Startposition (Fig. 4 a) bzw. in der Stopposition (Fig. 4 b) zeigen, wobei die Zeichenebene senkrecht zur Rotationsachse 14 verläuft. In dieser Startposition schließt eine durch die Rotationsachse 14 und die Strahlenquelle S definierte Ebene mit der Vertikalen einen Winkel α (entgegen der durch einen Pfeil P angedeuteten Umlaufrichtung) ein, der dem halben Öffnungswinkel des Strahlenbündels entspricht. Dabei wird von dem aus dem eingangs genannten Dokument bekannten Rekonstruktionsverfahren ausgegangen, das mit einem Rebinning arbeitet, bei dem zueinander und zur Rotationsachse parallele Strahlenfächer verarbeitet werden.

Demgemäss kann man sich das von der Strahlenquelle S in der Startposition emittierte Strahlenbündel in eine Anzahl von zur Rotationsachse parallelen Strahlenfächern zerlegt denken, von denen in Fig. 4 a die Strahlenfächer 41, 42, 43 dargestellt sind. Davon werden die gestrichelt angedeuteten Strahlenfächer 41 und 42 für die Rekonstruktion nicht ausgenutzt. Von dem in einer vertikalen Ebene verlaufenden Strahlenfächer 43 wird lediglich der in Richtung der Relativbewegung am weitesten vorne liegende Strahl ausgenutzt

Im weiteren Verlauf der Relativbewegung der Strahlenquelle werden neben mindestens einem Strahl aus einem Strahlenfächer in einer vertikalen Ebene (die aber gegenüber der Ebene des Strahlenfächers 43 nach links versetzt ist) auch Strahlen in Strahlenfächern zur Rekonstruktion herangezogen, die nicht in einer vertikalen Ebene liegen. Nach einem Umlauf der Strahlenquelle um den Winkel 2α um die Rotationsachse 14 ist schon ein Satz von in vertikalen Ebenen liegenden Strahlenfächern emittiert worden, die den Untersuchungsbereich 13 in seiner gesamten Breite durchsetzen. (es werden auch Strahlenfächer in anderen Ebenen emittiert; aber die in vertikalen Ebenen liegenden Strahlenfächer durchsetzen als erste den Untersuchungsbereich in seiner gesamten Breite.)

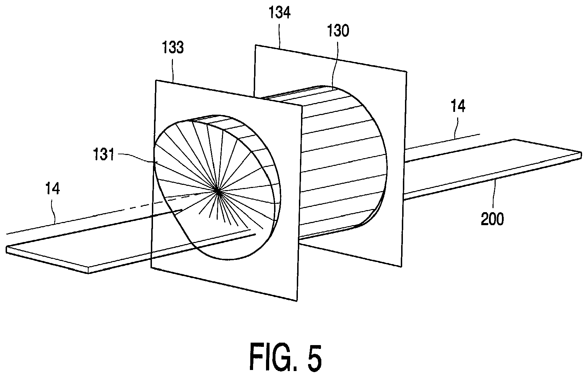

Diese vertikalen Strahlenfächer enthalten an ihrem in Vorschubrichtung vorderen Rand Strahlen, die von der jeweiligen Strahlenquellenposition auf einem Teilstück der Helix ausgehen und auf ein jenseits des Untersuchungsbereiches befindliches Teilstück der Helix treffen. Diese Strahlen definieren die Stirnfläche 131, die das rekonstruierbare Volumen auf der einen Seite begrenzt. Der Winkel, den diese Strahlen mit der Vertikalen einschließen, ist ortsabhängig; jedoch sind die Endpunkte der Strahlen gegenüber ihren Anfangspunkten in Richtung der Relativbewegung versetzt. Infolgedessen ergibt sich die in Fig. 5 dargestellte Form der Stirnfläche 131, so dass hier der obere Teil des Bereichs 130 über den unteren hinausragt.

Fig. 4b erläutert die Verhältnisse in der Stopposition, in der die Ebene, die durch die Strahlenquelle S und die Rotationsachse definiert ist, mit der Vertikalen einen Winkel α in Umlaufrichtung einschließt. Auch von den in dieser Position erzeugten Strahlen wird nur noch einer benötigt, der sich in dem in einer vertikalen Ebene liegenden Strahlenfächer 44 befindet. Die Strahlen aus den anderen Strahlenfächern 45 und 46 bzw. die zugehörigen CT-Daten werden zur Rekonstruktion nicht mehr benötigt. Somit wird auch die Stirnfläche 132, die den Bereich 130 auf der anderen Seite abschließt, durch Strahlen gebildet, die in vertikalen Ebenen verlaufen. Auch hierbei ragt die Stirnfläche oben weiter nach außen als unten.

Wenn man darauf verzichtet, den in- oder unterhalb der Lagerungsplatte 200 befindlichen Teil des Untersuchungsbereichs vollständig zu rekonstruieren, ist es möglich, die beiden Grenzebenen 133 und 134 weiter nach außen zu verschieben, wie ein Vergleich zwischen Fig. 3 und Fig. 5 zeigt. Aber auch wenn man sich nicht auf senkrechte Schichten beschränkt, ergeben sich bei Fig. 5 günstigere Verhältnisse, weil der rekonstruierbare Teil des Untersuchungsbereichs oberhalb der Lagerungsplatte 200 größer ist als unterhalb.

Um dem Untersucher auch für diese nicht rekonstruierbaren Teile die gewohnte Darstellung zu bieten, könnte man hier die Absorptionsverteilung in weiter im Innern des Bereichs 130 liegenden Schichten zugrundelegen. Es wäre jedoch auch möglich, die nicht rekonstruierbaren Bereiche in oder unterhalb der Lagerungsplatte in einer Darstellung farbig zu markieren.

Aufgrund der Tatsache, dass die Stirnflächen nicht eben sind, sind die anhand von Fig. 4 a und 4 b erläuterten Startpositionen nur für eine ganz bestimmte Höhe der Lagerungsplatte 200 optimal, nämlich dann, wenn die Oberseite der Lagerungsplatte 200 mit der Rotationsachse 14 zusammenfällt. Wenn die Lagerungsplatte höher oder tiefer eingestellt ist, gilt dies nur näherungsweise.

Die Fig. 6 a und 6b stellen eine Draufsicht auf die Stirnflächen 131 bzw. 132 dar (wobei die Betrachtungsrichtung in beiden Fällen gleich ist), mit denen das rekonstruierbare Volumen beginnt bzw. endet. Der Pfeil P stellt die Umlaufrichtung der Strahlenquelle dar. Auf der Stirnfläche 131 sind Linien Z1, Z2 .. Z5 .. Z7 und Z8 eingezeichnet, die die Punkte auf den Stirnflächen miteinander verbinden, die dieselbe z-Koordinate haben. Die Linie Z5 wird von der Rotationsachse 14 durchstoßen. Sie ist exakt gerade und horizontal. Die Linien darunter, wie z. B. die Linien Z1 und Z2, fallen nach rechts hin ab, während die Linien darüber, wie z. B. Z7 und Z8, nach rechts hin ansteigen. Diese Linien sind nur näherungsweise geradlinig.

Optimale Verhältnisse ergeben sich dann, wenn die Oberseite der Lagerungsplatte mit einer dieser Linien zusammenfällt. Befindet sich die Lagerungsplatte also unterhalb der Rotationsachse, dann müsste entweder die Lagerungsplatte etwas gekippt sein, was nicht möglich ist, oder die Stirnfläche müsste aus der in Fig. 6 a dargestellten Position etwas in Richtung des Pfeiles P gedreht sein. Dies würde voraussetzen, dass die Stirnfläche 131 nicht mehr durch Strahlen aus vertikalen Strahlenfächern definiert wäre, sondern durch Strahlen aus zueinander parallelen Strahlenfächern, die mit der Vertikalen einen kleinen Winkel einschließen, so dass z. B. die Höhenlinie Z2 horizontal verläuft. Das wiederum würde voraussetzen, dass die Startposition nicht so liegt wie in Fig. 4 a dargestellt, sondern bei einem Winkel in bezug auf die Vertikale, der kleiner ist als α.

Fig. 6b zeigt entsprechend Linien Z1', Z2' .. Z5' .. Z7' und Z8'. Man erkennt, dass diese Linien spiegelverkehrt zu den Linien Z1 .. Z8 auf der Stirnfläche 131 verlaufen, d. h. dass unterhalb der Rotationsachse befindlichen Linien nach rechts hin ansteigen und oberhalb der Rotationsachse befindlichen Linien nach rechts hin abfallen. Dementsprechend muss die Stirnfläche 132 entgegen der Umlaufrichtung des Pfeiles P gedreht werden, wenn die Lagerungsplatte sich unterhalb der Rotationsachse befindet. Dies setzt voraus, dass die Stopposition etwas entgegen der Umlaufrichtung verschoben wird, und zwar im gleichen Maße wie auch die Startposition in Umlaufrichtung verschoben werden muss. Auch die Stopposition liegt also nicht so wie in Fig. 4b dargestellt, sondern bei einem Winkel in bezug auf die Vertikale, der kleiner ist als α.

Um die Start- und/oder die Stopposition automatisch an die jeweilige Höhe der Lagerungsplatte 200 anzupassen, wird die Höhe, in die der Motor 6 die Platte 200 verschiebt (vgl. Fig. 1), der Steuereinheit 7 zugeführt, die in Abhängigkeit von der jeweiligen Höhe die optimale Start- und Stopposition bestimmt.

Die Lagerungsplatte 200 wird proportional zur Größe des Winkels β in z-Richtung verschoben. Wenn der Benutzer die Verschiebung in z-Richtung aber vorgibt, dann kann die aus der optimalen Startposition gestartete Strahlenquelle in der Regel nicht in der optimalen Stopposition enden, sondern in einer anderen Position. In diesem Fall ergibt sich nur für die Startfläche 131 die gewünschte räumliche Lage, bzw. nur für die Stirnfläche 132 (für den Fall, dass man Startposition so wählt, dass sich bei dem gewünschten Vorschub in z-Richtung am Ende gerade die optimale Stopposition ergibt). Die optimale Lage des Rekonstruktionsbereichs 130 ergibt sich dann nur zu Beginn bzw. am Ende des Umlaufs. Um dies zu vermeiden, kann man die Wahl des Vorschubes in z-Richtung auch so gestalten, dass nur die Anzahl n der Umdrehungen vorgegeben wird.

## Patentansprüche

1. Computertomograph mit:
- einer Abtasteinheit, die eine Strahlenquelle (S) und eine damit verbundene Detektoreinheit (16) zur Erfassung eines von der Strahlenquelle (S) emittierten, kegelförmigen Strahlenbündels (4) nach dem Durchgang durch einen Untersuchungsbereich (13) bzw. durch ein darin auf einer Lagerungsplatte (200) gelagertes Objekt und zur Erzeugung entsprechender CT-Daten umfasst,
- einer Antriebsanordnung (2,5) zur Erzeugung einer eine Rotation um eine Rotationsachse (14) und einen Vorschub parallel zur Rotationsachse umfassenden Relativbewegung in Form einer Helix zwischen der Abtasteinheit (8,16) und dem Untersuchungsbereich (13) bzw. dem Objekt,
- einer Rekonstruktionseinheit (10) zur Rekonstruktion eines dreidimensionalen CT-Bildes aus den von der Detektoreinheit (16) erzeugten Messwerten, und
- einer Steuereinheit (7) zur Steuerung der Strahlenquelle (S) und der Antriebsanordnung (2, 5) in der Weise, dass die Emission des Strahlenbündels (4) durch die Strahlenquelle (S) in einer solchen Startposition beginnt und/oder in einer solchen Stoppposition endet, dass die Bereiche, die zu Beginn und/oder am Ende der Emission vom Strahlenbündel in einem für die Rekonstruktion nicht hinreichenden Maße getroffen werden, unterhalb der Lagerungsplatte (200) in Richtung der Rotationsachse(14) größer sind als oberhalb der Lagerungsplatte,
wobei die Strahlenquelle (S) in der Startposition mit der Rotationsachse (14) eine Ebene definiert, die mit einer senkrechten Ebene durch die Rotationsachse (14) einen Winkel α entgegen der Umlaufrichtung (P) der Strahlenquelle einschließt, der dem halben Öffnungswinkel des Strahlenbündels (4) in einer zur Rotationsachse (14) senkrechten Ebene entspricht oder kleiner ist als der halbe Öffnungswinkel, und
wobei die Strahlenquelle (S) in der Stoppposition mit der Rotationsachse (14) eine Ebene definiert, die mit einer senkrechten Ebene durch die Rotationsachse (14) einen Winkel α in Umlaufrichtung (P) der Strahlenquelle (S) einschließt, der dem halben Öffnungswinkel des Strahlenbündels (4) in einer zur Rotationsachse (14) senkrechten Ebene entspricht oder kleiner ist als der halbe Öffnungswinkel.

2. Computertomograph nach Anspruch 1 mit Mitteln zur Erfassung der vertikalen Position der Lagerungsplatte (200) und mit Mitteln zur Veränderung der Startposition und/oder der Stoppposition in Abhängigkeit von der vertikalen Position der Lagerungsplatte (200).

3. Computertomograph nach Anspruch 1, wobei die Rekonstruktionseinheit (10) so programmiert ist, dass für die Bereiche in oder unterhalb der Lagerungsplatte (200), die zu Beginn und/oder am Ende der Emission vom Strahlenbündel (4) in einem für die Rekonstruktion nicht hinreichenden Maße getroffen werden, die aus einer vollständigen Rekonstruktion resultierende Absorptionsverteilung von in Richtung der Rotationsachse (14) benachbarten Schichten zugrundegelegt wird.

## Claims

1. Computer tomograph with:
- a scanning unit, which comprises a radiation source (S) and a detector unit (16) connected to the latter for detecting a conical beam (4) emitted by the radiation source (S) after passing through an examination area (13) or through an object placed in the latter on a support plate (200) and for generating corresponding CT data,
- a drive arrangement (2, 5) for generating a relative movement including a rotation about an axis of rotation (14) and a feed movement parallel to the axis of rotation in the form of a helix between the scanning unit (8,16) and the examination area (13) or the object,
- a reconstruction unit (10) for reconstructing a three-dimensional CT image from the readings produced by the detector unit (16),
- a control unit (7) for controlling the radiation source (S) and the drive arrangement (2, 5) in such a way that the emission of the beam (4) by the radiation source (S) begins at such a start position and/or ends at such a stop position that the areas, which at the beginning and/or end of the emission of the beam are not hit by the beam in a manner sufficient for reconstruction, are larger below the support plate (200) in the direction of the axis of rotation (14) than above the support plate,
wherein the radiation source (S) in the start position defines a plane with the axis of rotation (14) which forms an angle α with a perpendicular plane through the axis of rotation (14) against the circumferential direction (P) of the radiation source, which corresponds to half the beam angle of the beam (4) in a plane perpendicular to the axis of rotation (14) or is smaller than half the beam angle, and
wherein the radiation source (S) in the stop position defines a plane with the axis of rotation (14) which forms an angle α with a perpendicular plane through the axis of rotation (14) in circumferential direction (P) of the radiation source (S) which corresponds to half the beam angle of the beam (4) in a plane perpendicular to the axis of rotation (14) or is smaller than half the beam angle.

2. Computer tomograph according to claim 1 comprising means for detecting the vertical position of the support plate (200) and means for changing the start position and/or stop position as a function of the vertical position of the support plate (200).

3. Computer tomograph according to claim 1, wherein the reconstruction unit (10) is programmed so that for the areas in or below the support plate (200), which at the beginning and/or at the end of the emission of the beam (4) are not hit in a manner sufficient for reconstruction, the distribution of absorption resulting from a complete reconstruction is determined by layers which are adjacent in the direction of the axis of rotation (14).

## Revendications

1. Tomodensimètre avec :
- une unité de balayage qui comporte une source de rayons (S) et une unité de détection (16) reliée à celle-ci pour la détection d'un faisceau de rayons (4) en forme de cône émis par la source de rayons (S) après le passage par une zone d'examen (13) ou par un objet logé dedans sur une plaque de support (200) et pour la génération de données CT correspondantes,
- un agencement d'entraînement (2, 5) pour la génération d'un mouvement relatif comprenant une rotation autour d'un axe de rotation (14) et une avance parallèle à l'axe de rotation sous la forme d'une hélice entre l'unité de balayage (8, 16) et la zone d'examen (13) ou l'objet,
- une unité de reconstruction (10) pour la reconstruction d'une image CT tridimensionnelle à partir des valeurs de mesure générées par l'unité de détection (16), et
- une unité de commande (7) pour la commande de la source de rayons (S) et l'agencement d'entraînement (2, 5) de telle manière que l'émission du faisceau de rayons (4) par la source de rayons (S) commence dans une telle position de départ et/ou se termine dans une telle position d'arrêt que les zones qui sont atteintes au début et/ou à la fin de l'émission du faisceau de rayons dans une mesure insuffisante pour la reconstruction, soient plus grandes en dessous de la plaque de support (200) en direction de l'axe de rotation (14) qu'au-dessus de la plaque de support,
dans lequel la source de rayons (S) dans la position de départ avec l'axe de rotation (14) définit un plan qui forme avec un plan perpendiculaire au travers de l'axe de rotation (14) un angle α dans le sens inverse au sens périphérique (P) de la source de rayons qui correspond au demi-angle d'ouverture du faisceau de rayons (4) dans un plan perpendiculaire à l'axe de rotation (14) ou est inférieur au demi-angle d'ouverture, et
dans lequel la source de rayons (S) dans la position d'arrêt avec l'axe de rotation (14) définit un plan qui forme avec un plan perpendiculaire au travers de l'axe de rotation (14) un angle α dans le sens périphérique (P) de la source de rayons (S) qui correspond au demi-angle d'ouverture du faisceau de rayons (4) dans un plan perpendiculaire à l'axe de rotation (14) ou est inférieur au demi-angle d'ouverture.

2. Tomodensimètre selon la revendication 1 avec des moyens pour la détection de la position verticale de la plaque de support (200) et avec des moyens pour la modification de la position de départ et/ou de la position d'arrêt en fonction de la position verticale de la plaque de support (200).

3. Tomodensimètre selon la revendication 1, dans lequel l'unité de reconstruction (10) est programmée de sorte que pour les zones dans ou en dessous de la plaque de support (200) qui sont atteintes au début et/ou à la fin de l'émission du faisceau de rayons (4) dans une mesure insuffisante pour la reconstruction, la distribution d'absorption résultant d'une reconstruction complète de couches contiguës en direction de l'axe de rotation (14) est prise pour base.
